# EUROPEAN PATENT APPLICATION

(11) **EP 4 201 949 A1**
(43) Date of publication of application: **28.06.2023**
(21) Application number: 20950065.1
(22) Date of filing: 30.10.2020
(51) Int. Cl.: C07H 21/04, C07H 21/00

(54) **AMMONOLYSIS SOLUTION AND AMMONOLYSIS METHOD**

(30) Priority: 18.08.2020 CN 202010828506
(71) Applicant: GENEWIZ INC., SZ, Jiangsu 215123 (CN)
(72) Inventor: CAO, Chunyan, Suzhou, Jiangsu 215123 (CN); ZHAO, Shuang, Suzhou, Jiangsu 215123 (CN); JIN, Kaibao, Suzhou, Jiangsu 215123 (CN); JI, Lei, Suzhou, Jiangsu 215123 (CN); HAN, Chenghao, Suzhou, Jiangsu 215123 (CN); ZHU, Zhihao, Suzhou, Jiangsu 215123 (CN)
(74) Representative: Cabinet Laurent & Charras
(86) International application number: PCT/CN2020/125164
(87) International publication number: WO 2022/036862

(57) **Abstract**

An ammonolysis solution and an ammonolysis method. The ammonolysis solution comprises amine, butanol, amino alcohol, and an alkaline aqueous solution. The ammonolysis method comprises: (1) adding an ammonolysis solution to a solid-phase synthesis column where a solid phase carrier is contained; (2) placing the solid-phase synthesis column into a sealed container where an ammonolysis buffer solution is contained, and keeping the solid-phase synthesis column from contacting with the ammonolysis buffer solution; (3) heating the sealed container, so that the solid phase carrier is under a steam atmosphere of the ammonolysis buffer solution, and then cooling; and (4) taking the solid-phase synthesis column out of the sealed container, treating the solid phase carrier in the solid-phase synthesis column using an eluent, and collecting an effluent liquid. The ammonolysis solution is mild in formula. The ammonolysis buffer solution not only provides an ammonolysis atmosphere, but also assists in an ammonolysis reaction. The ammonolysis process does not use toxic gases, is safe and environmentally friendly, and does not damage the primer structure; meanwhile, the post-treatment steps are simple, cross contamination is avoided, and the large-scale industrial application can be achieved.

## Description

### TECHNICAL FIELD

The present application belongs to the technical field of synthesis of nucleic acids, and relates to an aminolysis solution and an aminolysis method.

### BACKGROUND

Short-stranded DNA, which is also known as an oligonucleotide (oligo for short), is generally synthesized artificially by using a solid-phase synthesis technique. During solid-phase synthesis, with a controlled pore glass (CPG) sphere as a solid phase carrier and phosphoramidite as a monomer, a target sequence is gradually ligated to the solid phase carrier in a 3'-5' direction after cycles of four reactions including deprotection, condensation, oxidation and capping. The solid-phase synthesis method has the following advantages: each reaction step is carried out on the solid phase carrier so that unreacted raw materials can be removed through suction filtration after the reaction is completed, and post-treatment steps are simple. Therefore, the solid-phase synthesis is widely used for oligo and polypeptide synthesis. In most of the current oligo synthesis processes, 5'-OH of the phosphoramidite monomer is protected by a dimethoxytriphenyl (DMT) group and may be cleaved in a 2-3% CCl₃COOH/CH₂Cl₂ solution, which is referred to as a deprotection reaction; free hydroxyl exposed after deprotection undergo a nucleophilic substitution reaction with phosphoramidite, i.e., a condensation reaction; after the condensation reaction is completed, unreacted free hydroxyl need to be acetylated to prevent unreacted hydroxyl from entering the next condensation reaction, which is referred to as "capping"; finally, being very unstable, phosphoramidite needs to be oxidized into +5 valent phosphorus with an oxidizing agent such as an iodine solution. Four reactions are carried out for each base ligation. After target bases are all synthesized, the oligo is cleaved from CPG. Meanwhile, since active amino groups contained in A, C and G bases interfere with the condensation reaction, the amino groups are generally protected with acetyl (Ac), benzoyl (Bz) and N,N-dimethyl formamidine (dmf) in advance, and Ac, Bz and dmf groups are cleaved after the reaction is completed. Here, the reaction of cleaving oligo from CPG and removing protecting groups is referred to as aminolysis reaction. An aminolysis reaction mainly includes: (1) cleaving oligo from the solid phase carrier; (2) exposing 3'-OH of the oligo; (3) cleaving protecting groups Ac, Bz and dmf on the A, C and G bases; and (4) cleaving cyanoethyl on phosphoric acid.

The aminolysis reaction is carried out by using aqueous ammonia at first. Its problems mainly include a long reaction time, where 16 hours are needed for the reaction at 55 °C and 8 hours are needed for the reaction at 65 °C; in the reaction process, a synthesis column needs to be soaked in aqueous ammonia, easily causing cross-contamination; since the oligo can be dissolved in aqueous ammonia, the post-treatment steps are complicated; and impurities in aqueous ammonia accumulate, causing pollution to the oligo. The aminolysis reaction system including methylamine : aqueous ammonia = 1: 1 (AMA) and developed later greatly reduces the reaction time (M.P. Reddy, N.B. Hanna, and F. Farooqui, Nucleos. Nucleot., 1997, 16, 1589-1598), but the problems of cross-contamination and complicated post-treatment steps still exist. Gas-phase aminolysis uses ammonia gas as an active medium and uses a high pressure resistant aminolysis pot as a reaction vessel to carry out the aminolysis reaction under the reaction conditions of a high temperature and a high pressure. The gas-phase aminolysis has the advantages of a quick and efficient reaction and simple operations, but exists the shortcomings of a high equipment requirement, a need to use a toxic gas, a potential safety hazard, environmental pollution and a relatively great damage to the structure of a primer. Microwave aminolysis is a process widely applied in recent years, which mainly uses microwaves as a heating means. However, in an actual operation process, microwave heating easily causes uneven heating, and continuous heating will cause liquid bumping, causing a loss and cross-contamination.

CN109956987A discloses a method for aminolysis after solid-phase synthesis of DNA. The method includes the following steps: (1) adding an aminolysis composition to a synthesis column including a solid phase carrier for synthesizing a DNA fragment; (2) placing the synthesis column in a sealable container containing an aminolysis buffer solution and maintaining the synthesis column above the surface of the aminolysis buffer solution; (3) microwaving the sealable container such that the solid phase carrier is under a vapor atmosphere of the aminolysis buffer solution; and (4) taking the synthesis column out, treating the solid phase carrier with an eluent, and collecting an effluent. However, the method still has the problems of uneven microwave heating and easy to cause a damage or cross-contamination.

Therefore, it is necessary to develop a new aminolysis method, which has mild reaction conditions, does not damage the structure of a primer, and solves the problems existing in the existing art.

### SUMMARY

The present application provides an aminolysis solution and an aminolysis method. The aminolysis solution is mild in formula. An aminolysis buffer solution not only provides an aminolysis atmosphere but also assists in an aminolysis reaction. An aminolysis process does not use a toxic gas, has high safety, is environmentally friendly, and does not damage the structure of a primer. Meanwhile, post-treatment steps are simple and avoid cross- contamination.

In a first aspect, the present application provides an aminolysis solution. The aminolysis solution includes amine, butanol, amino alcohol and an alkaline aqueous solution.

In the present application, the amine cooperates with two types of alcohol to cleave a synthetic oligonucleotide from a solid phase carrier and remove protecting groups Ac, Bz and dmf on A, C and G bases, wherein the amine functions as an active component of the aminolysis solution, butanol functions as a reaction solvent, and amino alcohol functions as both the active component and the reaction solvent.

In the present application, a trace amount of the alkaline aqueous solution is added to the aminolysis solution to function as a catalyst, and cooperates with the amine, butanol and amino alcohol, helping to accelerate an aminolysis reaction rate. The alkaline aqueous solution catalyzes aminolysis and does not affect the yield and quality of the oligonucleotide.

Preferably, the amine includes propylamine and/or butylamine.

Preferably, propylamine includes n-propylamine and/or isopropylamine.

Preferably, butylamine includes any one or a combination of at least two of n-butylamine, isobutylamine, sec-butylamine or tert-butylamine, preferably n-butylamine.

In the present application, the used propylamine and/or butylamine function as active components of the aminolysis solution, and cooperate with butanol, amino alcohol and the alkaline aqueous solution to exert efficient aminolysis activity and implement an efficient aminolysis reaction at a particular ratio.

Preferably, butanol includes any one or a combination of at least two of n-butanol, isobutanol, sec-butanol or tert-butanol, preferably n-butanol.

Preferably, amino alcohol includes amino butanol and/or amino pentanol.

Preferably, amino pentanol includes 5-amino-1-pentanol and/or 4-amino-1-pentanol, preferably 5-amino-1-pentanol.

Preferably, the alkaline aqueous solution includes any one or a combination of at least two of an aqueous solution of LiOH, an aqueous solution of NaOH, an aqueous solution of KOH, an aqueous solution of Be(OH)₂, an aqueous solution of Mg(OH)₂ or an aqueous solution of Ca(OH)₂.

Preferably, the alkaline aqueous solution has a concentration of 1-10 M, which may be, for example, 1 M, 2 M, 3 M, 4 M, 5 M, 6 M, 7 M, 8 M, 9 M or 10 M, preferably 5 M.

In the present application, the concentration of the alkaline aqueous solution is limited to a range of 1-10 M, which facilitates an efficient aminolysis reaction. The concentration of the alkaline aqueous solution cannot be too low or too high. Too low a concentration cannot promote the aminolysis reaction. Too high a concentration affects the yield and purity of the oligonucleotide, where the oligonucleotide obtained through elution easily generates a salt peak during mass spectrometry and the dried oligonucleotide may appear white.

Preferably, a volume ratio of the amine, butanol, amino alcohol and the alkaline aqueous solution is (5-10):(1-5):1:(0.01-0.1), which may be, for example, 5:1:1:0.01, 5:2:1:0.05, 5:5:1:0.1, 6:1:1:0.01, 6:2:1:0.05, 6:5:1:0.1, 7:1:1:0.01, 7:2:1:0.05, 7:5:1:0.1, 8:1:1:0.01, 8:2:1:0.05, 8:5:1:0.1, 9:1:1:0.01, 9:2:1:0.05, 9:5:1:0.1, 10:1:1:0.01, 10:2:1:0.05 or 10:5:1:0.1, preferably (6-8):(2-3):1:(0.04-0.06).

In the present application, the amine, butanol, amino alcohol and the alkaline aqueous solution cooperate with each other at a particular ratio, the amine, butanol and amino alcohol, as the main functional components of the aminolysis solution, cleave the synthetic oligonucleotide from the solid phase carrier and remove the protecting groups Ac, Bz and dmf on the A, C and G bases, and a trace amount of the alkaline aqueous solution catalyzes aminolysis and not only improves the yield of the oligonucleotide but also prevent the oligonucleotide from appearing white. The obtained oligonucleotide has high purity.

Preferably, a volume ratio of n-butylamine, n-butanol, 5-amino-1-pentanol and an aqueous solution of NaOH/KOH is (5-10):(1-5):1:(0.01-0.1), preferably (6-8):(2-3):1:(0.04-0.06).

In the present application, a ratio of the alkaline aqueous solution in the aminolysis solution cannot be too low or too high, too low a ratio cannot promote the aminolysis reaction, and too high a ratio affects the yield and purity of the oligonucleotide.

In a second aspect, the present application provides an aminolysis buffer solution. The aminolysis buffer solution includes aqueous ammonia and/or an organic amine.

In the present application, the aminolysis buffer solution is vaporized into vapor under a heating condition, which provides a suitable aminolysis environment, helping to improve aminolysis efficiency. When aqueous ammonia is used as the aminolysis buffer solution, the vaporized ammonia can not only provide the suitable aminolysis environment but also assist in liquid-phase aminolysis to conduct a gas-phase aminolysis reaction, thereby significantly improving the aminolysis efficiency and the yield of an oligonucleotide and shortening a reaction time.

Preferably, the organic amine includes any one or a combination of at least two of diethylamine, ethylenediamine or pyridine.

In the present application, when the organic amine is used as the aminolysis buffer solution, the vaporized organic amine can not only provide the suitable aminolysis environment but also assist in the liquid-phase aminolysis to conduct the gas-phase aminolysis reaction, thereby significantly improving the aminolysis efficiency and the yield of the oligonucleotide and shortening the reaction time.

In a third aspect, the present application provides an aminolysis method. The method includes the following steps:
(1) adding the aminolysis solution in the first aspect to a solid phase synthesis column containing a solid phase carrier;
(2) placing the solid phase synthesis column in a sealed container containing the aminolysis buffer solution in the second aspect and maintaining the solid phase synthesis column in non-contact with the aminolysis buffer solution;
(3) heating the sealed container such that the solid phase carrier is under a vapor atmosphere of the aminolysis buffer solution and then cooling;
(4) taking the solid phase synthesis column out of the sealed container, treating the solid phase carrier in the solid phase synthesis column with an eluent, and collecting an effluent.

In the present application, the solid phase carrier is soaked in the aminolysis solution in the first aspect so that an aminolysis reaction can occur under a mild heating condition, the aminolysis buffer solution in the sealed container is vaporized to promote the occurrence and progress of the aminolysis reaction, a synthetic oligonucleotide is cleaved from the solid phase carrier, and amino protecting groups on bases are removed, which is conducive to obtaining an oligonucleotide product with high purity and a high yield.

Preferably, in step (1), the solid phase carrier includes a controlled pore glass sphere.

According to the present application, a gap exists between spheres of the controlled pore glass (CPG) sphere and the aminolysis solution flows in the gap to carry out the aminolysis reaction so that the oligonucleotide is cleaved from CPG.

Preferably, in step (1), the solid phase carrier is ligated with the synthetic oligonucleotide. The oligonucleotide is an artificially synthesized DNA short strand and generally composed of 20-60 bases and may be used as a primer, a sequencing linker, a probe, or the like. The aminolysis solution of the present application can cleave the artificially synthesized DNA short strand from the solid phase carrier and cleave amino protecting groups Ac, Bz and dmf on bases A, C and G.

Preferably, in step (1), the solid phase carrier is soaked in the aminolysis solution.

Preferably, in step (2), the sealed container includes a sealed metal box.

In the present application, the sealed metal box is used as an aminolysis box to provide an atmosphere slightly higher than atmospheric pressure, and metal is a good conductor of heat, facilitating the rapid transfer of the heating temperature provided by a heating device to the inside of the aminolysis box and efficiently initiating the vaporization of the aminolysis buffer solution and the occurrence of the aminolysis reaction. Meanwhile, the sealed aminolysis box effectively avoids the losses of the aminolysis solution and the aminolysis buffer solution due to volatilization, facilitating the efficient completion of the aminolysis reaction.

Preferably, the temperature for the heating in step (3) makes the aminolysis buffer solution vaporized to form the vapor atmosphere of the aminolysis buffer solution in the sealed container so that the solid phase synthesis column is under the vapor atmosphere of the aminolysis buffer solution, so as to promote an aminolysis effect of the aminolysis solution and cleave the synthetic oligonucleotide from the solid phase carrier. The heating is preferably conducted at a temperature of 45-100 °C, which may be, for example, 45 °C, 50 °C, 55 °C, 60 °C, 65 °C, 70 °C, 75 °C, 80 °C, 81 °C, 82 °C, 83 °C, 84 °C, 85 °C, 86 °C, 87 °C, 88 °C, 89 °C, 90 °C, 91 °C, 92 °C, 93 °C, 94 °C, 95 °C, 96 °C, 97 °C, 98 °C, 99 °C or 100 °C, further preferably 90-95 °C.

Preferably, a time for the heating in step (3) is related to a synthesis specification, a formulation of the aminolysis solution, the heating temperature, a volume of the sealed container and a desired degree of completion of the aminolysis reaction and adjusted according to specific implementation situations. The heating is preferably conducted for 30-60 min, which may be, for example, 30 min, 35 min, 40 min, 45 min, 50 min, 55 min or 60 min.

Preferably, in step (3), the cooling includes an ice bath for 5-15 min, preferably an ice bath for 10 min.

Preferably, in step (4), the eluent includes water and/or Tris-HCl.

Preferably, Tris-HCl has a concentration of 10-30 mM, which may be, for example, 10 mM, 11 mM, 12 mM, 13 mM, 14 mM, 15 mM, 16 mM, 17 mM, 18 mM, 19 mM, 20 mM, 21 mM, 22 mM, 23 mM, 24 mM, 25 mM, 26 mM, 27 mM, 28 mM, 29 mM or 30 mM, preferably 25 mM.

Preferably, Tris-HCl has a pH of 7-8, which may be, for example, 7, 7.1, 7.2, 7.3, 7.4, 7.5, 7.6, 7.7, 7.8, 7.9 or 8, preferably 7.2.

Preferably, before the solid phase carrier in the solid phase synthesis column is treated with the eluent in step (4), a step of drying and washing the solid phase synthesis column is further included.

Preferably, the drying is conducted at 50-60 °C for 1-5 min to remove droplets of the aminolysis buffer solution condensed on a surface of the solid phase synthesis column. The temperature for the drying may be, for example, 50 °C, 51 °C, 52 °C, 53 °C, 54 °C, 55 °C, 56 °C, 57 °C, 58 °C, 59 °C or 60 °C, preferably 55 °C. The time for the drying may be, for example, 1 min, 2 min, 3 min, 4 min or 5 min, preferably 3 min.

Preferably, a washing liquid for the washing includes 100% acetonitrile and/or 80-95% acetonitrile for removing an unreacted reagent and a reaction byproduct.

Preferably, the washing includes washing the dried solid phase synthesis column with 100% acetonitrile and 80-95% acetonitrile in sequence.

Preferably, after the solid phase synthesis column is washed, a step of centrifugation is further included.

As a preferred technical solution, the present application provides an aminolysis method. The method includes the following steps:
(1) adding the aminolysis solution in the first aspect to a solid phase synthesis column containing a controlled pore glass sphere ligated with a synthetic oligonucleotide and soaking the controlled pore glass sphere;
(2) placing the solid phase synthesis column in a sealed metal container containing an aminolysis buffer solution and maintaining the solid phase synthesis column in non-contact with the aminolysis buffer solution, where the aminolysis buffer solution includes aqueous ammonia and/or an organic amine;
(3) heating the sealed metal container at 45-100 °C for 30-60 min such that the controlled pore glass sphere ligated with the synthetic oligonucleotide is under a vapor atmosphere of the aminolysis buffer solution and then cooling in an ice bath for 5-15 min;
(4) taking the solid phase synthesis column out of the sealed container, drying the solid phase synthesis column at 50-60 °C for 1-5 min, washing the dried solid phase synthesis column with 100% acetonitrile and 80-95% acetonitrile in sequence, conducting centrifugation, treating the solid phase carrier in the solid phase synthesis column with water and/or 10-25 mM Tris-HCl with a pH of 7-8, and collecting an effluent.

Compared with the existing art, the present application has the beneficial effects described below.
(1) The aminolysis solution of the present application is concise in formulation, where butylamine, butanol and amino pentanol cooperate with each other to cleave the synthetic oligonucleotide from the solid phase carrier and remove the protecting groups Ac, Bz and dmf on the bases A, C and G, and the aminolysis solution is further added with the alkaline aqueous solution to accelerate the rate of the aminolysis reaction.
(2) The aminolysis reaction of the present application is conducted in the sealed metal aminolysis box, and the aminolysis buffer solution inside the metal aminolysis box is vaporized under the heating condition to provide the suitable aminolysis environment for the aminolysis reaction. As the aminolysis buffer solution, aqueous ammonia not only provides the suitable aminolysis environment but also has the effect of gas-phase aminolysis and can assist in a liquid-phase aminolysis reaction.
(3) The aminolysis process of the present application has an important application value due to mild conditions, a low cost, environmental friendliness and high safety.

### BRIEF DESCRIPTION OF DRAWINGS

FIG. 1 shows an HPLC spectrum of an oligonucleotide having a length of 23 bases.
FIG. 2 shows an ESI mass spectrum of an oligonucleotide having a length of 23 bases.
FIG. 3 shows an HPLC spectrum of an oligonucleotide having a length of 60 bases.
FIG. 4 shows an ESI mass spectrum of an oligonucleotide having a length of 60 bases.
FIG. 5 shows an HPLC spectrum of an oligonucleotide having a length of 86 bases.
FIG. 6 shows an ESI mass spectrum of an oligonucleotide having a length of 86 bases.

### DETAILED DESCRIPTION

To further elaborate on the technical means adopted and effects achieved in the present application, the present application is further described below in conjunction with examples and drawings. It is to be understood that the specific examples described herein are intended to explain the present application and not to limit the present application.

Experiments without specific techniques or conditions specified in the examples are conducted according to techniques or conditions described in the literature in the art or a product specification. The reagents or instruments used herein without manufacturers specified are conventional products commercially available from proper channels.

### Example 1

In this example, a primer having a length of 23 bases, a primer having a length of 60 bases and a primer having a length of 86 bases were subjected to an aminolysis reaction separately, whose sequences were as follows:
SEQ ID NO: 1 (23 nt) with a target molecular weight of 7030.6:
   5'-CATTCGTAGCTCGGATCGTGTAC-3';
SEQ ID NO: 2 (60 nt) with a target molecular weight of 18270.8: 
5’-
GCATGGCGACCTTATCAGTCTACGTCCTTTGTCTTCCTAAGACCGCTTGGCCTCCGAC
TT-3’;
SEQ ID NO: 3 (86 nt) with a target molecular weight of 26543.2:

5’-
GCCTGCTTGAGCAGGCTGAAGTTGGTGGCACGCGTTTAATTAACCATATCATCTAGTG
TTGCATATGAGACAAGGACTTTTCCCAT-3’.

Steps are described below.

### Primer synthesis

A 50 nmol synthesis column was used, an acetonitrile solution containing 0.25 M 5-ethylthiotetrazole (ETT) was used as an activator, a dichloromethane solution containing 3% trichloroacetic acid was used as a deprotecting reagent, 10% acetic anhydride/acetonitrile and N-methylimidazole/pyridine/acetonitrile (with a ratio of 14:10:76) were used as capping reagents, tetrahydrofuran/pyridine/water (with a ratio of 70:20:10) containing 0.05 M iodine was used as an oxidizing agent, and a synthesis reaction was conducted on a Dr. Oligo 192 synthesizer.

### Liquid-phase aminolysis

(1) 20 µL of an aminolysis solution was added to a solid phase synthesis column and CPG ligated with a synthesized primer was soaked therein, where the formulation of the aminolysis solution was n-butylamine : n-butanol : 5-amino-1-pentanol : an aqueous solution of NaOH (5 M) with a volume ratio of 6:3:1:0.05.
(2) 20 mL of aqueous ammonia was poured into a metal aminolysis box with a specification of 170 mm × 130 mm × 95 mm.
(3) A synthetic plate including multiple solid phase synthesis columns was carefully placed into the aminolysis box and stood still for 1 min, and the metal box was covered tightly and fixed and sealed by screws to ensure that the solid phase synthesis columns were in non-contact with an aminolysis buffer solution.
(4) The metal aminolysis box was carefully placed into an oven, heated at 90 °C for 60 min, taken out after the reaction was completed, and cooled in an ice bath for 10 min.
(5) The metal aminolysis box was taken out, the screws were loosened, and the synthetic plate was taken out, dried in an oven at 55 °C for 3 min, and cooled outside the oven to room temperature.
(6) The synthetic plate was washed twice with 100% acetonitrile, 100 µL of acetonitrile each time, and washed once with 100 µL of 90% acetonitrile/water.
(7) The synthetic plate was taken out and centrifuged at 3000 rpm for 1 min.
(8) 100 µL of 25 mM Tris-HCl eluent with a pH of 7.2 was added for elution, and an effluent was collected for mass spectrometry.

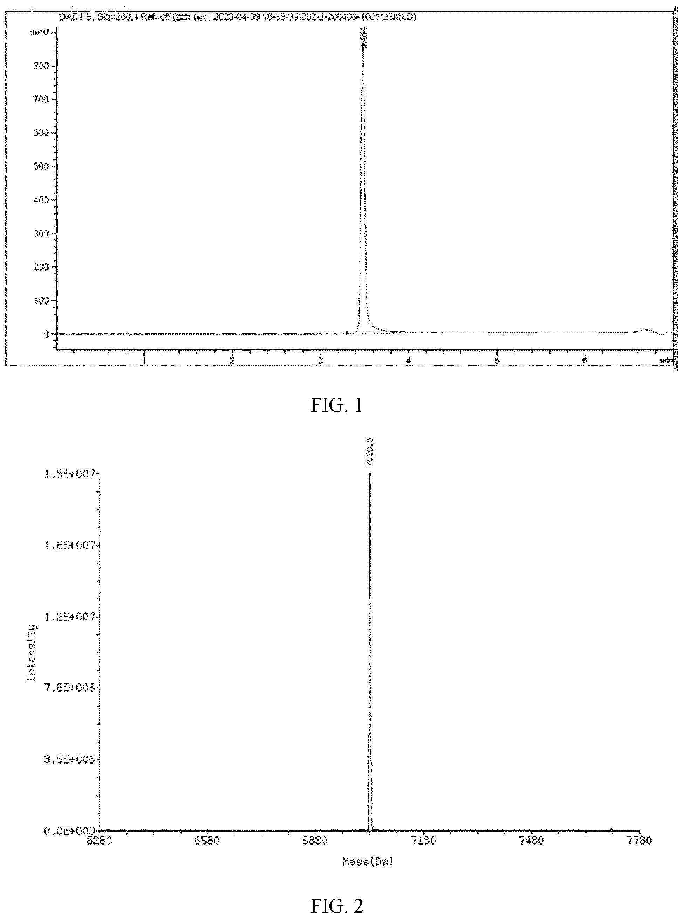

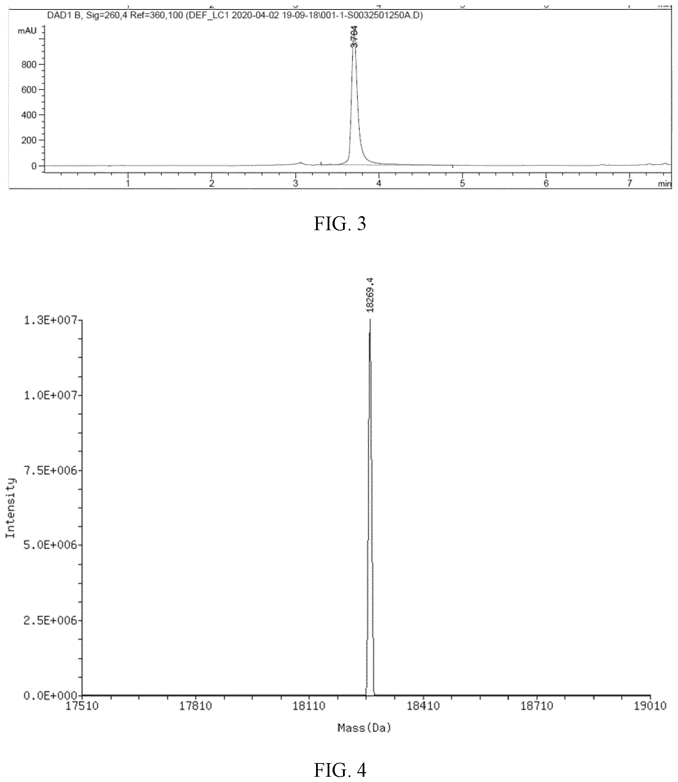

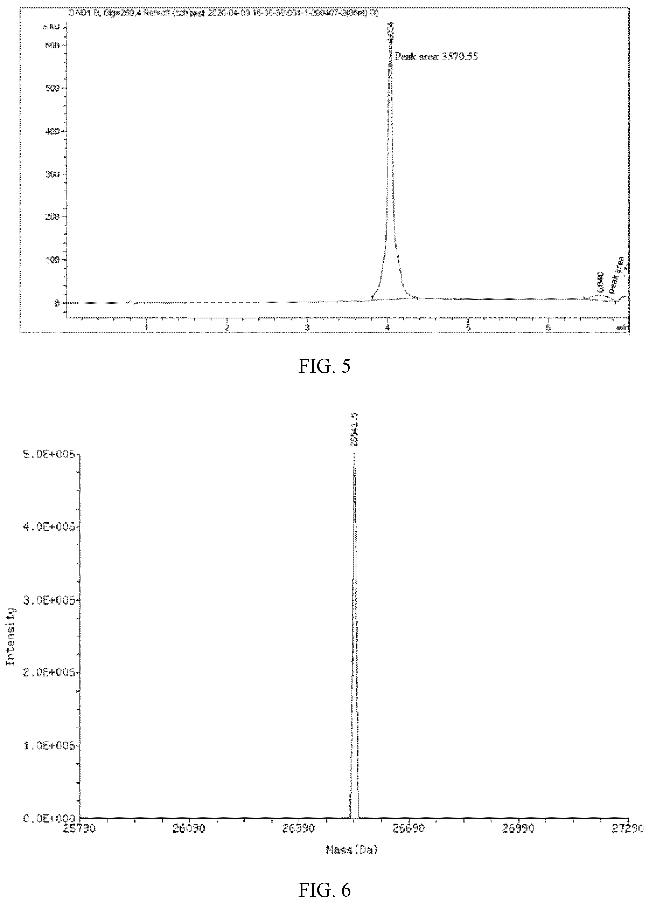

The HPLC spectrum and ESI mass spectrum of the primer having a length of 23 bases, the primer having a length of 60 bases and the primer having a length of 86 bases are shown in FIGS. 1, 2, 3, 4, 5 and 6, respectively. It can be seen that the HPLC purity and mass spectra of the all primers having different lengths and subjected to the liquid-phase aminolysis can reach industrial standards, proving that the aminolysis method of the present application has relatively good aminolysis effects on oligonucleotides with different lengths.

### Example 2

In this example, 96 primers each having a length of 40-60 bases were subjected to aminolyses by the aminolysis method in Example 1 to analyze a qualified rate of mass spectrometry and a yield.

Steps are described below.

### Primer synthesis

A 5 nmol synthesis column was used, an acetonitrile solution containing 0.25 M 5-ethylthio tetrazole (ETT) was used as an activator, a dichloromethane solution containing 3% trichloroacetic acid was used as a deprotecting reagent, 10% acetic anhydride/acetonitrile and N-methylimidazole/pyridine/acetonitrile (with a ratio of 14:10:76) were used as capping reagents, tetrahydrofuran/pyridine/water (with a ratio of 70:20:10) containing 0.05 M iodine was used as an oxidizing agent, and a synthesis reaction was conducted on a Dr. Oligo 768 synthesizer.

### Liquid-phase aminolysis

(1) 20 µL of an aminolysis solution was added to a solid phase synthesis column and CPG ligated with a synthesized primer was soaked therein, where the formulation of the aminolysis solution was n-butylamine : n-butanol : 5-amino-1-pentanol : an aqueous solution of NaOH (5 M) with a volume ratio of 7:2:1:0.05.
(2) 20 mL of aqueous ammonia was poured into a metal aminolysis box with a specification of 170 mm × 130 mm × 95 mm.
(3) A synthetic plate including multiple solid phase synthesis columns was carefully placed into the aminolysis box and stood still for 1 min, and the metal box was covered tightly and fixed and sealed by screws to ensure that the solid phase synthesis columns were in non-contact with an aminolysis buffer solution.
(4) The metal aminolysis box was carefully placed into an oven, heated at 90 °C for 60 min, taken out after the reaction was completed, and cooled in an ice bath for 10 min.
(5) The metal aminolysis box was taken out, the screws were loosened, and the synthetic plate was taken out, dried in an oven at 55 °C for 3 min, and cooled outside the oven to room temperature.
(6) The synthetic plate was washed twice with 100% acetonitrile, 200 µL of acetonitrile each time, and washed once with 200 µL of 90% acetonitrile/water.
(7) The synthetic plate was taken out and centrifuged at 3000 rpm for 1 min.
(8) 200 µL of 25 mM Tris-HCl eluent with a pH of 7.2 was added for elution, and an effluent was collected for mass spectrometry and microplate reader quantification.

### Gas-phase aminolysis

(1) The off-machine synthetic plate was directly placed into a gas phase aminolysis pot and reacted at 90 °C for 40 min under 80-100 Mpsi.
(2) The synthetic plate was carefully taken out of the aminolysis pot, dried in an oven at 55 °C for 3 min, and cooled outside the oven to room temperature.
(3) The synthetic plate was washed twice with 100% acetonitrile, 200 µL of acetonitrile each time, and washed once with 200 µL of 90% acetonitrile/water.
(4) The synthetic plate was taken out and centrifuged at 3000 rpm for 1 min.
(5) 200 µL of 25 mM Tris-HCl eluent with a pH of 7.2 was added for elution, and an effluent was collected for mass spectrometry and microplate reader quantification.

The yields and purity of the primers of the liquid-phase aminolysis and the gas-phase aminolysis are shown in Table 1. It can be seen that compared with the gas-phase aminolysis, the liquid-phase aminolysis method of the present application obtains 96 primers at a higher average yield; with a mass spectrometry purity of 75% as a standard for qualification, the qualified rate of mass spectrometry of the liquid-phase aminolysis is slightly higher than that of the gas-phase aminolysis. It indicates that the liquid-phase aminolysis method of the present application improves the primer and purity of an oligonucleotide compared with the conventional gas-phase aminolysis.

**Table 1 Average yield and purity of 96 primers**

| Aminolysis Method | Yield (nmol) | Purity (%) |
|---|---|---|
| Gas-phase aminolysis | 3.26 | 96.9 |
| Liquid-phase aminolysis | 3.59 | 98.9 |

Table 2 shows the comparison between the liquid-phase aminolysis and the gas-phase aminolysis in NGS purity and cross-contamination. It can be seen that the oligonucleotide prepared through the liquid-phase aminolysis has high purity, a low intraplate cross-contamination rate and a low interplate cross-contamination rate.

**Table 2**

| Liquid-phase aminolysis | | | | Gas-phase aminolysis | | | |
|---|---|---|---|---|---|---|---|
| Sampl e No. | Purity | Intraplate Contamina tion | Interplate Contamina tion | Sampl e No. | Purit y | Intraplate Contamina tion | Interplate Contamina tion |
| Liqui d phase -E1 | 99.98 % | 0.02% | 0.00% | Gas phase -B1 | 99.97 % | 0.02% | 0.01% |
| Liqui d phase -H1 | 99.99 % | 0.01% | 0.00% | Gas phase -E1 | 99.98 % | 0.02% | 0.00% |
| Liqui d phase -A2 | 100.00 % | 0.00% | 0.00% | Gas phase -H1 | 99.95 % | 0.03% | 0.00% |
| Liqui d phase -B2 | 100.00 % | 0.00% | 0.00% | Gas phase -A2 | 99.84 % | 0.16% | 0.00% |
| Liqui d phase -F2 | 99.99 % | 0.01% | 0.00% | Gas phase -B2 | 99.99 % | 0.01% | 0.00% |
| Avera ge value | 99.99 % | 0.04% | 0.00% | Avera ge value | 99.95 % | 0.05% | 0.00% |

### Example 3

In this example, 96 primers each having a length of 40-60 bases were synthesized by using a 50 nmol synthesis column. For synthesis steps, refer to Example 2. A qualified rate of mass spectrometry and a yield were analyzed after aminolyses. Steps are described below.
(1) 20 µL of an aminolysis solution was added to a solid phase synthesis column and CPG ligated with a synthesized primer was soaked therein, where the formulation of the aminolysis solution was isobutylamine : n-butanol : 5-amino-1-pentanol : an aqueous solution of KOH (6 M) with a volume ratio of 6:2:1:0.04.
(2) 20 mL of aqueous ammonia was poured into a metal aminolysis box with a specification of 170 mm × 130 mm × 95 mm.
(3) A synthetic plate including multiple solid phase synthesis columns was carefully placed into the aminolysis box and stood still for 1 min, and the metal box was covered tightly and fixed and sealed by screws to ensure that the solid phase synthesis columns were in non-contact with an aminolysis buffer solution.
(4) The metal aminolysis box was carefully placed into an oven, heated at 95 °C for 40 min, taken out after the reaction was completed, and cooled in an ice bath for 10 min.
(5) The metal aminolysis box was taken out, the screws were loosened, and the synthetic plate was taken out, dried in an oven at 55 °C for 3 min, and cooled outside the oven to room temperature.
(6) The synthetic plate was washed twice with 100% acetonitrile, 200 µL of acetonitrile each time, and washed once with 200 µL of 90% acetonitrile/water.
(7) The synthetic plate was taken out and centrifuged at 3000 rpm for 1 min.
(8) 200 µL of 25 mM Tris-HCl eluent with a pH of 7.2 was added for elution, and an effluent was collected for mass spectrometry and microplate reader quantification.

### Example 4

In this example, 96 primers each having a length of 40-60 bases were synthesized by using a 50 nmol synthesis column. For synthesis steps, refer to Example 2. A qualified rate of mass spectrometry and a yield were analyzed after aminolyses. Steps are described below.
(1) 20 µL of an aminolysis solution was added to a solid phase synthesis column and CPG ligated with a synthesized primer was soaked therein, where the formulation of the aminolysis solution was n-propylamine : isobutanol : 4-amino-1-pentanol : LiOH (4 M) with a volume ratio of 6:2:1:0.06.
(2) 20 mL of diethylamine was poured into a metal aminolysis box with a specification of 170 mm × 130 mm × 95 mm.
(3) A synthetic plate including multiple solid phase synthesis columns was carefully placed into the aminolysis box and stood still for 1 min, and the metal box was covered tightly and fixed and sealed by screws to ensure that the solid phase synthesis columns were in non-contact with an aminolysis buffer solution.
(4) The metal aminolysis box was carefully placed into an oven, heated at 85 °C for 50 min, taken out after the reaction was completed, and cooled in an ice bath for 10 min.
(5) The metal aminolysis box was taken out, the screws were loosened, and the synthetic plate was taken out, dried in an oven at 55 °C for 3 min, and cooled outside the oven to room temperature.
(6) The synthetic plate was washed twice with 100% acetonitrile, 200 µL of acetonitrile each time, and washed once with 200 µL of 90% acetonitrile/water.
(7) The synthetic plate was taken out and centrifuged at 3000 rpm for 1 min.
(8) 200 µL of 20 mM Tris-HCl eluent with a pH of 7.2 was added for elution, and an effluent was collected for mass spectrometry and microplate reader quantification.

### Example 5

In this example, 96 primers each having a length of 40-60 bases were synthesized by using a 50 nmol synthesis column. For synthesis steps, refer to Example 2. A qualified rate of mass spectrometry and a yield were analyzed after aminolyses. Steps are described below.
(1) 20 µL of an aminolysis solution was added to a solid phase synthesis column and CPG ligated with a synthesized primer was soaked therein, where the formulation of the aminolysis solution was sec-butylamine : isobutanol : 4-amino-1-pentanol : an aqueous solution of Be(OH)₂ (2 M) with a volume ratio of 8:3:1:0.05.
(2) 20 mL of diethylamine was poured into a metal aminolysis box with a specification of 170 mm × 130 mm × 95 mm.
(3) A synthetic plate including multiple solid phase synthesis columns was carefully placed into the aminolysis box and stood still for 1 min, and the metal box was covered tightly and fixed and sealed by screws to ensure that the solid phase synthesis columns were in non-contact with an aminolysis buffer solution.
(4) The metal aminolysis box was carefully placed into an oven, heated at 100 °C for 30 min, taken out after the reaction was completed, and cooled in an ice bath for 10 min.
(5) The metal aminolysis box was taken out, the screws were loosened, and the synthetic plate was taken out, dried in an oven at 55 °C for 3 min, and cooled outside the oven to room temperature.
(6) The synthetic plate was washed twice with 100% acetonitrile, 200 µL of acetonitrile each time, and washed once with 200 µL of 80% acetonitrile/water.
(7) The synthetic plate was taken out and centrifuged at 3000 rpm for 1 min.
(8) 200 µL of pure water elute was added for elution, and an effluent was collected for mass spectrometry and microplate reader quantification.

### Example 6

In this example, 96 primers each having a length of 40-60 bases were synthesized by using a 50 nmol synthesis column. For synthesis steps, refer to Example 2. A qualified rate of mass spectrometry and a yield were analyzed after aminolyses. Steps are described below.
(1) 20 µL of an aminolysis solution was added to a solid phase synthesis column and CPG ligated with a synthesized primer was soaked therein, where the formulation of the aminolysis solution was tert-butylamine : sec-butanol : amino butanol : an aqueous solution of Mg(OH)₂ (1 M) with a volume ratio of 5: 1: 1:0.1.
(2) 20 mL of ethylenediamine was poured into a metal aminolysis box with a specification of 170 mm × 130 mm × 95 mm.
(3) A synthetic plate including multiple solid phase synthesis columns was carefully placed into the aminolysis box and stood still for 1 min, and the metal box was covered tightly and fixed and sealed by screws to ensure that the solid phase synthesis columns were in non-contact with an aminolysis buffer solution.
(4) The metal aminolysis box was carefully placed into an oven, heated at 60 °C for 35 min, taken out after the reaction was completed, and cooled in an ice bath for 10 min.
(5) The metal aminolysis box was taken out, the screws were loosened, and the synthetic plate was taken out, dried in an oven at 55 °C for 3 min, and cooled outside the oven to room temperature.
(6) The synthetic plate was washed twice with 100% acetonitrile, 200 µL of acetonitrile each time, and washed once with 200 µL of 95% acetonitrile/water.
(7) The synthetic plate was taken out and centrifuged at 3000 rpm for 1 min.
(8) 200 µL of pure water eluent was added for elution, and an effluent was collected for mass spectrometry and microplate reader quantification.

### Example 7

In this example, an aminolysis reaction was conducted by using a formulation of an aminolysis solution with a different volume ratio. Mass spectrometry and yields were compared to analyze the effects of aminolysis components on an aminolysis effect. Specific experimental steps are described below.
(1) 20 µL of an aminolysis solution was added to a solid phase synthesis column and CPG ligated with a synthesized primer was soaked therein, where the formulation of the aminolysis solution was isopropylamine : tert-butanol : amino butanol : Ca(OH)₂ (10 M) with a volume ratio of 10:5:1:0.01.
(2) 20 mL of pyridine was poured into a metal aminolysis box with a specification of 170 mm × 130 mm × 95 mm.
(3) A synthetic plate including multiple solid phase synthesis columns was carefully placed into the aminolysis box and stood still for 1 min, and the metal box was covered tightly and fixed and sealed by screws to ensure that the solid phase synthesis columns were in non-contact with an aminolysis buffer solution.
(4) The metal aminolysis box was carefully placed into an oven, heated at 45 °C for 35 min, taken out after the reaction was completed, and cooled in an ice bath for 10 min.
(5) The metal aminolysis box was taken out, the screws were loosened, and the synthetic plate was taken out, dried in an oven at 55 °C for 3 min, and cooled outside the oven to room temperature.
(6) The synthetic plate was washed twice with 100% acetonitrile, 200 µL of acetonitrile each time, and washed once with 200 µL of 95% acetonitrile/water.
(7) The synthetic plate was taken out and centrifuged at 3000 rpm for 1 min.
(8) 200 µL of pure water eluent was added for elution, and an effluent was collected for mass spectrometry and microplate reader quantification.

### Comparative Example 1

Compared with that in Example 2, the formulation of an aminolysis solution was diethylenetriamine : an aqueous solution of LiOH (1 M) : ethanol with a volume ratio of 1:3:6, and other conditions were the same as those in Example 2. Mass spectrometry and yields were compared to analyze the effects of aminolysis components on an aminolysis effect. Specific experimental steps are the same as those in Example 2.

The yields and purity of the primers in Examples 2 to 7 and Comparative Example 1 are shown in Table 3, which indicate that the aminolysis formulations can ensure both the yields and purity of low-yield primers (synthesized by 5 nmol) and high-yield primers (synthesized by 50 nmol) and have a wide application range; the functional components diethylenetriamine and ethanol of the aminolysis solution used in Comparative Example 1 have relatively weak aminolysis activity, so an alkaline aqueous solution with a high ratio needs to be added to ensure the occurrence of the aminolysis reaction, which causes the problem of a poor primer quality: the primers are seriously whitened and have relatively many impurities, and complicated post-treatment steps affect the structures and the functions of the primers.

**Table 3 Average yield and purity of 96 primers under different aminolysis conditions**

| No. | Yield (nmol) | Purity (%) | Appearance |
|---|---|---|---|
| Example 2 | 3.59 | 98.9 | No whitening |
| Example 3 | 35.26 | 98.5 | No whitening |
| Example 4 | 36.60 | 99.4 | No whitening |
| Example 5 | 33.49 | 97.6 | No whitening |
| Example 6 | 35.45 | 98.8 | No whitening |
| Example 7 | 34.03 | 98.0 | No whitening |
| Comparative Example 1 | 33.22 | 97.8 | Whitening |

In conclusion, the aminolysis solution of the present application is concise in formulation, low in cost, environmentally friendly and high in safety, and the aminolysis process has mild conditions so that the structure of the oligonucleotide is not damaged; the aminolysis reaction occurs in the sealed container so that the aminolysis solution and/or the aminolysis buffer solution are prevented from volatilization, facilitating the progress of the aminolysis reaction and achieving high aminolysis efficiency and simple and convenient operations; and the aminolysis buffer solution not only provides a suitable aminolysis environment but also has an effect of assisting in an aminolysis, further improving the aminolysis efficiency. The present application has an important application prospect in the field of solid-phase synthesis of nucleic acids.

The applicant states that although the detailed method of the present application is described through the examples described above, the present application is not limited to the detailed method described above, which means that the implementation of the present application does not necessarily depend on the detailed method described above. It should be apparent to those skilled in the art that any improvements made to the present application, equivalent replacements of raw materials of the product of the present application, additions of adjuvant ingredients, selections of specific manners, etc., all fall within the protection scope and the disclosure scope of the present application.

## Claims

1. An aminolysis solution, comprising amine, butanol, amino alcohol and an alkaline aqueous solution;
wherein a volume ratio of the amine, butanol, amino alcohol and the alkaline aqueous solution is (5-10):(1-5): 1 :(0.01-0.1).

2. The aminolysis solution according to claim 1, wherein the amine comprises propylamine and/or butylamine.

3. The aminolysis solution according to claim 2, wherein propylamine comprises n-propylamine and/or isopropylamine.

4. The aminolysis solution according to claim 2, wherein butylamine comprises any one or a combination of at least two of n-butylamine, isobutylamine, sec-butylamine or tert-butylamine.

5. The aminolysis solution according to claim 1, wherein butanol comprises any one or a combination of at least two of n-butanol, isobutanol, sec-butanol or tert-butanol.

6. The aminolysis solution according to claim 1, wherein amino alcohol comprises amino butanol and/or amino pentanol.

7. The aminolysis solution according to claim 6, wherein amino pentanol comprises 5-amino-1-pentanol and/or 4-amino-1-pentanol.

8. The aminolysis solution according to claim 1, wherein the alkaline aqueous solution comprises any one or a combination of at least two of an aqueous solution of LiOH, an aqueous solution of NaOH, an aqueous solution of KOH, an aqueous solution of Be(OH)₂, an aqueous solution of Mg(OH)₂ or an aqueous solution of Ca(OH)₂.

9. The aminolysis solution according to claim 1, wherein the alkaline aqueous solution has a concentration of 1-10 M.

10. The aminolysis solution according to claim 1, wherein the volume ratio of the amine, butanol, amino alcohol and the alkaline aqueous solution is (6-8):(2-3):1:(0.04-0.06).

11. The aminolysis solution according to claim 8, wherein the amine is n-butylamine, butanol is n-butanol, amino alcohol is 5-amino-1-pentanol, and a volume ratio of n-butylamine, n-butanol, 5-amino-1-pentanol and the alkaline aqueous solution is (5-10):(1-5):1:(0.01-0.1).

12. The aminolysis solution according to claim 11, wherein the volume ratio of n-butylamine, n-butanol, 5-amino-1-pentanol and the alkaline aqueous solution is (6-8):(2-3):1:(0.04-0.06).

13. An aminolysis method, comprising the following steps:
(1) adding the aminolysis solution according to any one of claims 1 to 12 to a solid phase synthesis column containing a solid phase carrier;
(2) placing the solid phase synthesis column in a sealed container containing an aminolysis buffer solution and maintaining the solid phase synthesis column in non-contact with the aminolysis buffer solution;
(3) heating the sealed container such that the solid phase carrier is under a vapor atmosphere of the aminolysis buffer solution and then cooling;
(4) taking the solid phase synthesis column out of the sealed container, treating the solid phase carrier in the solid phase synthesis column with an eluent, and collecting an effluent.

14. The method according to claim 13, wherein in step (1), the solid phase carrier comprises a controlled pore glass sphere.

15. The method according to claim 13, wherein in step (1), the solid phase carrier is ligated with a synthetic oligonucleotide.

16. The method according to claim 13, wherein in step (1), the solid phase carrier is soaked in the aminolysis solution.

17. The method according to claim 13, wherein in step (2), the sealed container comprises a sealed metal box.

18. The method according to claim 13, wherein in step (2), the aminolysis buffer solution comprises aqueous ammonia and/or an organic amine.

19. The method according to claim 18, wherein the organic amine comprises any one or a combination of at least two of diethylamine, ethylenediamine or pyridine.

20. The method according to claim 13, wherein in step (3), the heating is conducted at a temperature of 45-100 °C.

21. The method according to claim 13, wherein in step (3), the heating is conducted for 30-60 min.

22. The method according to claim 13, wherein in step (3), the cooling comprises an ice bath for 5-15 min.

23. The method according to claim 13, wherein in step (4), the eluent comprises water and/or Tris-HCl.

24. The method according to claim 23, wherein Tris-HCl has a concentration of 10-30 mM.

25. The method according to claim 24, wherein Tris-HCl has a pH of 7-8.

26. The method according to claim 13, before treating the solid phase carrier in the solid phase synthesis column with the eluent in step (4), further comprising a step of drying and washing the solid phase synthesis column.

27. The method according to claim 26, wherein the drying is conducted at 50-60 °C for 1-5 min.

28. The method according to claim 26, wherein a washing liquid for the washing comprises 100% acetonitrile and/or 80-95% acetonitrile.

29. The method according to claim 28, wherein the washing comprises washing the dried solid phase synthesis column with 100% acetonitrile and 80-95% acetonitrile in sequence.

30. The method according to claim 26, after washing the solid phase synthesis column, further comprising a step of centrifugation.

31. The method according to claim 13, comprising of the following steps:
(1) adding the aminolysis solution according to any one of claims 1 to 12 to a solid phase synthesis column containing a controlled pore glass sphere ligated with a synthetic oligonucleotide and soaking the controlled pore glass sphere;
(2) placing the solid phase synthesis column in a sealed metal container containing an aminolysis buffer solution and maintaining the solid phase synthesis column in non-contact with the aminolysis buffer solution, wherein the aminolysis buffer solution comprises aqueous ammonia and/or an organic amine;
(3) heating the sealed metal container at 45-100 °C for 30-60 min such that the controlled pore glass sphere ligated with the synthetic oligonucleotide is under a vapor atmosphere of the aminolysis buffer solution and then cooling in an ice bath for 5-15 min;
(4) taking the solid phase synthesis column out of the sealed container, drying the solid phase synthesis column at 50-60 °C for 1-5 min, washing the dried solid phase synthesis column with 100% acetonitrile and 80-95% acetonitrile in sequence, conducting centrifugation, treating the solid phase carrier in the solid phase synthesis column with water and/or 10-25 mM Tris-HCl with a pH of 7-8, and collecting an effluent.
